# EUROPEAN PATENT APPLICATION

(11) **EP 3 834 629 A1**
(43) Date of publication of application: **16.06.2021**
(21) Application number: 19846174.1
(22) Date of filing: 05.08.2019
(51) Int. Cl.: A23L 33/105, A23L 2/52, A61K 31/12, A61P 3/10

(54) **COMPOSITION AND METHOD FOR SUPPRESSING INCREASE IN BLOOD GLUCOSE LEVEL**

(30) Priority: 10.08.2018 JP 2018151540
(71) Applicant: Suntory Holdings Limited, Osaka 530-8203 (JP)
(72) Inventor: FUKIZAWA, Shinya, Soraku-gun, Kyoto 619-0284 (JP); NONAKA, Yuji, Soraku-gun, Kyoto 619-0284 (JP); YAMASHITA, Mai, Soraku-gun, Kyoto 619-0284 (JP)
(74) Representative: Vossius & Partner Patentanwälte Rechtsanwälte mbB
(86) International application number: PCT/JP2019/030711
(87) International publication number: WO 2020/031952

(57) **Abstract**

The present invention aims to provide a composition for suppressing an increase in blood glucose level, containing a thermally stable substance as an active ingredient; a composition for suppressing an increase in blood glucose level, the composition being capable of suppressing an increase in postprandial blood glucose level regardless of the timing of intake, whether before, during, or after meals; and a method of suppressing an increase in blood glucose level. The present invention relates to, for example, a composition for suppressing an increase in blood glucose level, containing isoxanthohumol as an active ingredient.

## Description

### TECHNICAL FIELD

The present invention relates to a composition for suppressing an increase in blood glucose level. The present invention also relates to a method of suppressing an increase in blood glucose level and use of isoxanthohumol for suppressing an increase in blood glucose level.

### BACKGROUND ART

Diabetes is a group of metabolic disorders mainly characterized by chronic hyperglycemic conditions due to lack of insulin action. A recent increase in the number of diabetic and prediabetic patients has been a social problem in terms of increased medical expenses mainly in developed and emerging countries. Large-scale clinical trials in the past and the like have demonstrated that lifestyle interventions such as dietary (total energy intake) restrictions and moderate exercise are effective approaches that lead to prevention of the onset of diabetes and amelioration of diabetes. However, forcing drastic lifestyle interventions to a subject for a long period of time imposes a physical burden on the subject. Thus, there is a demand for a method of preventing the onset of diabetes or a method of ameliorating diabetes which imposes less physical burden.

Chronic hyperglycemic conditions due to diabetes are the cause of characteristic complications. Thus, normal glycemic control is important in improving long-term prognosis, as described in Japanese Clinical Practice Guideline for Diabetes (The Japan Diabetes Society). Examples of indexes of glycemic control include fasting blood glucose level, postprandial blood glucose level, and HbA1c. According to a report, there is a positive correlation between blood glucose level two hours after sugar load and mortality (Non-Patent Literature 1). Correcting postprandial hyperglycemic conditions, i.e., suppressing an increase in blood glucose level after intake of a food containing carbohydrate, is considered to be clinically significant. As one of methods of preventing postprandial hyperglycemia, use of functional foods is considered to be beneficial in order to control medical expenses. For example, commercially available products include foods for specified health uses and foods with function claims which appeal an effect of suppressing an increase in postprandial blood glucose level. However, most of these products are designed to inhibit physical glucose absorption in the small intestine or to inhibit functions of digestive enzymes which break down polysaccharides and disaccharides into monosaccharides, and the timing of intake of these products is limited to before or during meals.

Xanthohumol is known as a component of hops (scientific name: *Humulus lupulus)* that are a plant of the family Cannabaceae and that are used as raw materials of beer. Xanthohumol is a polyphenol having a molecular formula represented by C₂₁H₂₂O₅, and is classified as "prenylchalcone" among polyphenols.

Non-Patent Literature 2 shows that continuous administration of xanthohumol at a dosage of 60 mg/kg/day to a high-fat diet mouse model decreases the fasting blood glucose level. It also reports that continuous administration of xanthohumol at a dosage of 30 or 60 mg/kg/day decreases the fasting blood insulin level. Non-Patent Literature 3 shows a decrease in blood glucose level in a high-fat diet mouse model after long-term breeding with xanthohumol (10 mg/L) mixed in drinking water. Patent Literature 1 shows a suppressed increase in blood glucose level in a glucose tolerance test in a diabetes mouse model after breeding with feed containing a high xanthohumol content material at a portion of 1%. The results shown in Non-Patent Literatures 2 and 3 and Patent Literature 1 are obtained as a result of continuous intake of xanthohumol. No reports have been made on an effect obtained by single intake of xanthohumol.

Xanthohumol, which has been reported to decrease the blood glucose level as described above, is irreversibly structurally converted into isoxanthohumol when heated. In particular, according to a report, a large portion of xanthohumol is converted into isoxanthohumol during beer brewing. In addition, substances and compositions that are particularly highly stable are desirable in foods for specified health uses, foods with function claims, and the like which require a guarantee of the amount of components involved in functionality during long-term storage.

According to a report, while xanthohumol and isoxanthohumol both have prenyl groups, the former has a chalcone skeleton as a partial structure and the latter has a flavanone skeleton as a partial structure, with different physicochemical properties and physiological activities therebetween. Non-Patent Literature 4 states that while xanthohumol has very effective anticancer action, isoxanthohumol hardly has anticancer action. Thus, it is difficult to analogize the physiological activity of isoxanthohumol based on information regarding the physiological activity of xanthohumol.

No reports have been made on isoxanthohumol that acts to suppress an increase in blood glucose level. Patent Literature 2 shows that xanthohumol and an extract of spent hops with absolute ethanol promote secretion of adiponectin from 3T3-L1 adipose cells. Adiponectin is a physiologically active substance that contributes to improving the insulin resistance (a condition with insufficient insulin action). Yet, Patent Literature 2 is silent about whether isoxanthohumol has an action that affects adiponectin secretion and about data from evaluation of the effect of isoxanthohumol on blood glucose level. In addition, according to the extraction conditions (40°C) described in the examples of Patent Literature 2, the temperature is not high enough to covert xanthohumol into isoxanthohumol. Thus, the amount of isoxanthohumol in the extract of hops with absolute ethanol is considered to be very small, and it is uncertain whether or not an action of isoxanthohumol is present.

### CITATION LIST

### - Patent Literature

Patent Literature 1: JP 2006-306800 A
Patent Literature 2: JP 2009-518429 T

### - Non-Patent Literature

Non-Patent Literature 1: Lancet 1999, 354; 617-621
Non-Patent Literature 2: Arch Biochem Biophys. 2016, 1; 599: 22-30
Non-Patent Literature 3: J Nutr Biochem. 2017, 45; 39-47.
Non-Patent Literature 4: Free Radical Bio Med. 2015, 89; 486-497

### SUMMARY OF INVENTION

### - Technical Problem

Based on the above social background, there have been demands for substances that can be used in foods and/or beverages, and methods, which are capable of suppressing postprandial hyperglycemia. Preferably, such substances in demand are those that can be used in foods for specified health uses, foods with function claims, and the like which require a guarantee of the amounts of components involved in functionality during long-term storage; that are thermally stable; and that contribute to suppressing an increase in blood glucose level. Preferably, such substances can also suppress an increase in postprandial blood glucose level, regardless of the timing of intake, whether before, during, or after meals.

The present invention aims to provide a composition for suppressing an increase in blood glucose level, containing a thermally stable substance as an active ingredient. The present invention also aims to provide a composition for suppressing an increase in blood glucose level, the composition being capable of suppressing an increase in postprandial blood glucose level regardless of the timing of intake, whether before, during, or after meals. The present invention also aims to provide, for example, a method of suppressing an increase in blood glucose level.

### - Solution to Problem

As a result of intensive studies to solve the above problems, the present inventors discovered that isoxanthohumol acts to suppress an increase in blood glucose level. Isoxanthohumol is a compound having a higher thermal stability than xanthohumol. Isoxanthohumol more significantly acted to suppress an increase in blood glucose level than xanthohumol. Single intake of isoxanthohumol does not exhibit the action to suppress an increase in blood glucose level. Continuous intake of isoxanthohumol is required for isoxanthohumol to exhibit the action to suppress an increase in blood glucose level. This indicates that isoxanthohumol does not physically inhibit absorption of glucose derived from carbohydrate. Instead, isoxanthohumol exhibits the effect of suppressing an increase in blood glucose level based on another mechanism of action. Thus, the timing of isoxanthohumol intake to obtain the effect of suppressing an increase in postprandial blood glucose level is not limited to before or during meals. Isoxanthohumol acts to suppress an increase in blood glucose level, and is highly suitable for a beverage. Thus, use of isoxanthohumol makes it possible to develop functional beverages and the like which contribute to maintaining and improving the health.

Specifically, the present invention relates to, but is not limited to, a composition for suppressing an increase in blood glucose level, a method of suppressing an increase in blood glucose level, and the like described below.
(1) A composition for suppressing an increase in blood glucose level, containing: isoxanthohumol as an active ingredient.
(2) The composition for suppressing an increase in blood glucose level according to (1) above, wherein the composition is used for suppressing an increase in postprandial blood glucose level.
(3) The composition for suppressing an increase in blood glucose level according to (1) or (2) above, wherein the composition is intended for continuous intake.
(4) The composition for suppressing an increase in blood glucose level according to any one of (1) to (3) above, wherein the composition is used for preventing or ameliorating a condition or disease caused by postprandial hyperglycemia.
(5) The composition for suppressing an increase in blood glucose level according to any one of (1) to (4) above, wherein the composition is a food or beverage.
(6) The composition for suppressing an increase in blood glucose level according to any one of (1) to (5) above, wherein the composition is a beverage.
(7) The composition for suppressing an increase in blood glucose level according to (6) above, wherein the beverage is a tea-based beverage, a coffee beverage, an alcohol beverage, a non-alcoholic beer-taste beverage, a carbonated beverage, a functional beverage, a fruit and/or vegetable-based beverage, a lactic beverage, a soy milk beverage, or flavored water.
(8) The composition for suppressing an increase in blood glucose level according to any one of (1) to (7) above, wherein the composition is labeled with one or more of the followings: "suppressing an increase in postprandial blood glucose level", "moderating increase in postprandial blood glucose level", "reducing blood glucose level", "for those who are concerned about blood glucose level", "for those who are concerned about postprandial blood glucose level", and "reducing the predisposition for hyperglycemia".
(9) A method of suppressing an increase in blood glucose level, including: feeding or administering isoxanthohumol to a subject.
(10) The method of suppressing an increase in blood glucose level according to (9) above, wherein isoxanthohumol is continuously fed or administered to the subject.
(11) Use of isoxanthohumol for suppressing an increase in blood glucose level.
(12) The use according to (11) above, wherein isoxanthohumol is continuously fed or administered to a subject.

### - Advantageous Effects of Invention

The present invention can provide a composition for suppressing an increase in blood glucose level, containing a thermally stable substance as an active ingredient. The present invention can also provide a composition for suppressing an increase in blood glucose level, the composition being capable of suppressing an increase in postprandial blood glucose level regardless of the timing of intake, whether before, during, or after meals. The present invention can provide a method of suppressing an increase in blood glucose level.

### BRIEF DESCRIPTION OF DRAWINGS

Fig. 1 is a graph showing fluctuations in blood glucose level of each group with single administration of isoxanthohumol or xanthohumol after glucose administration.
Fig. 2 is a graph showing fluctuations in blood glucose level of each group with continuous administration of isoxanthohumol or xanthohumol after glucose administration (#: p < 0.05 vs. high fat diet group).
Fig. 3 is a graph showing an area under the time curve (AUC) of blood glucose concentration of each group with continuous administration of isoxanthohumol or xanthohumol (++: p < 0.01 vs. regular diet group).

### DESCRIPTION OF EMBODIMENTS

The composition for suppressing an increase in blood glucose level of the present invention contains isoxanthohumol as an active ingredient.

As shown in examples described later, mice continuously fed (administrated) with isoxanthohumol showed a suppressed increase in blood glucose level after glucose intake, as compared to non-fed mice. Thus, isoxanthohumol acts to suppress an increase in blood glucose level. Isoxanthohumol intake can suppress an increase in postprandial blood glucose level. The effect of suppressing an increase in blood glucose level by isoxanthohumol can be obtained by continuous intake of isoxanthohumol. Thus, in the case of isoxanthohumol intake to obtain the effect of suppressing an increase in blood glucose level, e.g., an effect of suppressing an increase in postprandial blood glucose level, the timing of isoxanthohumol intake may be any timing without limitations, whether before, during, or after meals.

Isoxanthohumol can be prepared, for example, through a process such as heating of a hop *(Humulus lupulus)* extract. Heating of a hop extract can produce isoxanthohumol in the extract. A hop extract is usually prepared through a process involving extraction of hop cones with a solvent and purification as needed. A hop extract can be obtained by a known preparation method. Hops can be extracted, for example, by a method that uses an ethanol solvent, which is used as a preparation method of a hop extract for beer brewing. A hop extract is commercially available, and a commercial hop extract can also be used. A hop extract is heated to produce isoxanthohumol preferably at 80°C to 140°C (more preferably 85°C to 100°C) for 15 minutes to 5 hours (more preferably 20 minutes to 3 hours). The hop extract is purified to prepare isoxanthohumol by a known method. Purification is performed by, for example, a method using HPLC or an absorption column or a precipitation method based on changes in solubility. Isoxanthohumol can also be produced by isomerizing xanthohumol by heating. Here, the heating temperature is preferably 80°C to 140°C (more preferably 85°C to 100°C) for 15 minutes to 5 hours (more preferably 20 minutes to 3 hours). Isoxanthohumol obtained by isomerization can be concentrated or purified by a known method (e.g., filtration, vacuum concentration, or lyophilization), as needed.

Isoxanthohumol is a compound that is contained in natural products, foods, and/or beverages and that has been used in foods and beverages. Thus, daily intake of isoxanthohumol, for example, is unlikely to cause any problems in terms of safety. Thus, the present invention can provide a composition for suppressing an increase in blood glucose level, containing a highly safe component as an active ingredient, and a method of suppressing an increase in blood glucose level.

Isoxanthohumol has been reported to be stable even at a high temperature of 100°C, for example. The Food Sanitation Act defines sterilization conditions as the standard of soft drinks and the like. For example, soft drinks having a pH of 4.0 or higher (excluding those having a pH of 4.6 or higher and a water activity higher than 0.94) need to be heated at 85°C for 30 minutes. In view of conversion of ingredients in such a sterilization process, isoxanthohumol is considered to be highly suitable for beverage applications.

Thus, the present invention can provide various functional foods, functional beverages, and the like that act to suppress an increase in blood glucose level, that are thermally stable, and that contribute to maintenance and improvement of health. The present invention can also provide various functional foods, functional beverages, and the like that exhibit the effect of suppressing an increase in blood glucose level, such as an effect of suppressing an increase in postprandial blood glucose level, regardless of the timing of intake, whether before, during, or after meals.

Isoxanthohumol can be used for suppressing an increase in blood glucose level, for example, for suppressing an increase in postprandial blood glucose level. The composition for suppressing an increase in blood glucose level of the present invention contains isoxanthohumol as an active ingredient, so that the composition exhibits the effect of suppressing an increase in blood glucose level, and is suitably used, for example, to suppress an increase in postprandial blood glucose level. Use of the composition for suppressing an increase in blood glucose level of the present invention can prevent or ameliorate postprandial hyperglycemia. The composition for suppressing an increase in blood glucose level of the present invention can be used for preventing or ameliorating postprandial hyperglycemia, or can be used for preventing or ameliorating a condition or disease caused by postprandial hyperglycemia. Postprandial hyperglycemia refers to a symptom that shows increased blood glucose level after carbohydrate intake. Examples of the condition or disease caused by postprandial hyperglycemia include diabetes, arteriosclerosis, neuropathy, nephropathy, and retinopathy. The composition for suppressing an increase in blood glucose level of the present invention is useful for prevention or amelioration of such a condition or disease. Herein, the expression "prevention of a condition or disease" refers to preventing the onset of a condition or disease, delaying the onset of a condition or disease, reducing the incidence of a condition or disease, reducing the onset risk of a condition or disease, and the like. The expression "amelioration of a condition or disease" refers to helping a subject recover from a condition or disease, alleviating a symptom of a condition or disease, delaying or preventing the progress of a condition or disease, and the like.

The composition for suppressing an increase in blood glucose level of the present invention may contain isoxanthohumol as a sole active ingredient.

The composition for suppressing an increase in blood glucose level of the present invention is applicable for either therapeutic use (medical use) or non-therapeutic use (non-medical use).

The composition for suppressing an increase in blood glucose level of the present invention can be provided, for example, as a food or beverage, a pharmaceutical or quasi-pharmaceutical product, or feed. The composition for suppressing an increase in blood glucose level of the present invention may be a food or beverage, a pharmaceutical or quasi-pharmaceutical product, feed, or the like by itself for suppressing an increase in blood glucose level; or may be a material, a preparation, or the like to be added to such a product or the like.

For example, the composition for suppressing an increase in blood glucose level of the present invention may be provided as an agent or the like, but it is not limited thereto. The agent can be directly provided as a composition or can be provided as a composition containing the agent. The composition for suppressing an increase in blood glucose level of the present invention can also be referred to as an agent for suppressing an increase in blood glucose level.

Preferably, the composition for suppressing an increase in blood glucose level of the present invention is a composition for oral ingestion, in order to sufficiently obtain the effect of the present invention. The composition for oral ingestion may be a food or beverage, a pharmaceutical or quasi-pharmaceutical product for oral administration, or feed, preferably a food or beverage, more preferably a pharmaceutical product for oral administration, still more preferably a food or beverage.

The composition for suppressing an increase in blood glucose level of the present invention contains isoxanthohumol as an active ingredient. The composition for suppressing an increase in blood glucose level of the present invention can contain optional additives and optional components, in addition to isoxanthohumol, as long as the effect of the present invention is not impaired. Such additives and components can be selected according to the composition form or the like. Those that can be used generally in foods, beverages, pharmaceutical products, quasi-pharmaceutical products, feed, feed additives, and the like can be used.

When the composition for suppressing an increase in blood glucose level of the present invention is provided as a food or beverage, a pharmaceutical or quasi-pharmaceutical product, feed, or the like, any common method can be used for the production. The composition for suppressing an increase in blood glucose level which contains isoxanthohumol can also be produced, for example, by using a hop extract and heating the hop extract.

For example, the composition for suppressing an increase in blood glucose level of the present invention is provided as a food or beverage, a component usable in a food or beverage (e.g., a food material or an optional food additive) can be added to isoxanthohumol to provide various types of foods or beverages. Non-limiting examples of the food or beverage include general foods and beverages, health foods, health beverages, foods with function claims, foods for specified health uses, and foods and beverages for the sick. The health foods, the foods with function claims, and the foods for specified health uses can be used in various forms of preparations such as fine granules, tablets, granules, powders, capsules, chewable tablets, syrups, liquids, and liquid foods.

As described above, since isoxanthohumol is thermally stable, the composition for suppressing an increase in blood glucose level of the present invention can be provided in various beverage forms. In an exemplary preferred embodiment, the composition for suppressing an increase in blood glucose level of the present invention may be a beverage.

The beverage may be a non-alcohol beverage or an alcohol beverage. Examples of the non-alcohol beverage include tea-based beverages, coffee beverages, non-alcoholic beer-taste beverages, carbonated beverages, functional beverages, fruit and/or vegetable-based beverages, lactic beverages, soy milk beverages, and flavored water.

When the composition for suppressing an increase in blood glucose level of the present invention is a beverage, preferably, it is a tea-based beverage, a coffee beverage, an alcohol beverage, a non-alcoholic beer-taste beverage, a carbonated beverage, a functional beverage, a fruit and/or vegetable-based beverage, a lactic beverage, a soy milk beverage, or flavored water.

When the composition for suppressing an increase in blood glucose level of the present invention is a tea-based beverage, preferably, it is a black tea beverage or a sugarless tea beverage. Examples of the sugarless tea beverages include green tea beverages, oolong tea beverages, barley tea beverages, brown rice tea beverages, adlay tea beverages, and sugarless black tea beverages.

When the composition for suppressing an increase in blood glucose level of the present invention is a coffee beverage, preferably, it is a packaged coffee beverage or liquid coffee.

Examples of the alcohol beverage include beer, beer-based beverages, and alcohol beverages other than the beer and beer-based beverages.

When the composition for suppressing an increase in blood glucose level of the present invention is a beer-based beverage, preferably, it is low-malt beer or a beer-like beverage.

When the composition for suppressing an increase in blood glucose level of the present invention is an alcohol beverage other than the beer and beer-based beverages, preferably, it is *shochu,* a *shochu* highball, a liqueur, cocktail, a spirit, or a whisky.

The term "non-alcoholic beer-taste beverage" as used herein refers to carbonated beverages with beer-like flavors, which are usually non-fermented non-alcohol beverages, substantially free of alcohols. Here, the non-alcoholic beer-taste beverage does not exclude beverages containing a trace amount (undetectable amount) of alcohol.

When the composition for suppressing an increase in blood glucose level of the present invention is a carbonated beverage, preferably, it is a cola-flavored beverage, a clear carbonated beverage, ginger ale, a fruit juice-based carbonated beverage, a milk-containing carbonated beverage, or a sugarless carbonated beverage.

When the composition for suppressing an increase in blood glucose level of the present invention is a functional beverage, preferably, it is a sports drink, an energy drink, a health-supporting beverage, or a jelly drink pouch.

When the composition for suppressing an increase in blood glucose level of the present invention is a fruit and/or vegetable-based beverage, preferably, it is a 100% fruit juice, a fruit-containing beverage, a soft drink with a low fruit juice content, a pulp-containing fruit juice, or a pulp-containing beverage.

When the composition for suppressing an increase in blood glucose level of the present invention is a lactic beverage, preferably, it is milk, a yogurt drink, a lactic acid bacteria beverage, or a milk-containing soft drink.

When the composition for suppressing an increase in blood glucose level of the present invention is a soy milk beverage, preferably, it is soy milk or a soybean beverage.

The form of the beverage is not particularly limited. Examples include packaged beverages. Packages for the packaged beverages are not particularly limited. Packages in any form and of any material may be used. For example, any of the following commonly used packages can be used: metal packages such as aluminum cans and steel cans; resin containers such as plastic bottles; paper containers such as drink cartons; glass containers such as glass bottles; and wooden containers such as barrels. The beverage is filled and sealed in any of these packages, whereby a packaged beverage can be obtained.

When the composition for suppressing an increase in blood glucose level of the present invention is provided as a pharmaceutical or quasi-pharmaceutical product, various dosage forms of pharmaceutical or quasi-pharmaceutical products can be provided by, for example, adding a pharmacologically acceptable carrier, an optional additive, or the like to isoxanthohumol. Such a carrier, an additive, or the like may be of any pharmacologically acceptable type that can be used in pharmaceutical or quasi-pharmaceutical products. Examples thereof include excipients, binders, disintegrants, lubricants, antioxidants, and colorants. One or more of these can be used. The form of administration (intake) of the pharmaceutical or quasi-pharmaceutical product may be an oral, enteral, or transmucosal administration or injection. Oral administration is preferred in order to sufficiently obtain the effect of the present invention. When the composition for suppressing an increase in blood glucose level is a pharmaceutical product, preferably, it is a pharmaceutical product for oral administration. Examples of dosage forms of preparations for oral administration include liquids, tablets, powders, fine granules, granules, sugar-coated tablets, capsules, suspensions, emulsions, and chewable tablets. The dosage form of preparation for parenteral administration includes injections, inhalations, infusions, suppositories, percutaneous absorption agents, nasal drops, eye drops, creams, gels, and lotions. The pharmaceutical product may be a pharmaceutical product for non-human animals.

When the composition for suppressing an increase in blood glucose level of the present invention is provided as feed, for example, isoxanthohumol may be added to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of isoxanthohumol in the composition for suppressing an increase in blood glucose level of the present invention is not limited, and can be set according to the composition form or the like. For example, the amount of isoxanthohumol in the composition for suppressing an increase in blood glucose level is preferably 0.0001 wt% or more, more preferably 0.001 wt% or more, and is preferably 90 wt% or less. In one embodiment, the amount of isoxanthohumol in the composition for suppressing an increase in blood glucose level is preferably 0.0001 to 90 wt%, more preferably 0.001 to 90 wt%. In one embodiment, when the composition for suppressing an increase in blood glucose level of the present invention is provided as a food or beverage, a pharmaceutical or quasi-pharmaceutical product, feed, or the like, preferably, the amount of isoxanthohumol is in the above ranges.

Isoxanthohumol contained in the composition for suppressing an increase in blood glucose level can be quantified by, for example, high performance liquid chromatography (HPLC) or LC-MS/MS.

The composition for suppressing an increase in blood glucose level of the present invention can be fed or administered by an appropriate method according to the form. Preferably, the composition for suppressing an increase in blood glucose level of the present invention is orally fed (orally administered) in order to sufficiently obtain the effect of the present invention.

The intake (dosage) of the composition for suppressing an increase in blood glucose level of the present invention is not limited, as long as it is the amount (effective amount) that provides the effect of suppressing an increase in blood glucose level. The intake may be suitably set according to the dosage form, administration method, body weight of a subject, and the like. In one embodiment, when the composition for suppressing an increase in blood glucose level is orally fed or administered to a human (adult), the intake of the composition in terms of isoxanthohumol is preferably 1 to 200 mg, more preferably 5 to 60 mg, per 60 kg per day. Intake of the above amount in one or more portions per day, for example, one to several times (e.g., two to three times) per day, is preferred. The timing of intake of the composition for suppressing an increase in blood glucose level of the present invention is not limited. The composition may be fed or administered in one or more portions per day at any timing. In one embodiment, the composition for suppressing an increase in blood glucose level of the present invention may be a composition for oral administration to be used to feed the above amount of isoxanthohumol per 60 kg body weight per day to an adult.

A greater effect of suppressing an increase in blood glucose level is obtained by continuous intake (administration) of isoxanthohumol. Thus, in a preferred embodiment, the composition for suppressing an increase in blood glucose level of the present invention is fed continuously. In one embodiment of the present invention, the composition for suppressing an increase in blood glucose level is preferably fed continuously for two weeks or longer, more preferably three weeks or longer.

A subject to be fed (administered) with the composition for suppressing an increase in blood glucose level of the present invention is not limited. The subject is preferably a human or non-human mammal, more preferably a human.

The subject to be fed with the composition for suppressing an increase in blood glucose level of the present invention is preferably one who needs or wants to suppress an increase in blood glucose level, more preferably one who needs or wants to suppress an increase in postprandial blood glucose level. The subject to be fed with the composition for suppressing an increase in blood glucose level of the present invention is not limited to a diabetic or prediabetic patient. The composition for suppressing an increase in blood glucose level of the present invention can be used for therapeutic purposes to a hyperglycemic human, for example, whose fasting blood glucose is 126 mg/dL or higher or whose two-hour oral glucose tolerance test (OGTT) value is 200 mg/dL of higher. The composition for suppressing an increase in blood glucose level of the present invention can also be used for preventing exacerbation of diabetes of a human, for example, whose two-hour oral glucose tolerance test value is 140 to 199 mg/dL (borderline diabetic patient). The composition for suppressing an increase in blood glucose level of the present invention can also be used for preventing or ameliorating hyperglycemic conditions of a healthy person.

The composition for suppressing an increase in blood glucose level of the present invention contains, as an active ingredient, a highly safe component that is contained in natural products and/or foods or beverages. The composition for suppressing an increase in blood glucose level of the present invention exhibits the effect of suppressing an increase in blood glucose level when fed continuously. The timing of intake of the composition for suppressing an increase in blood glucose level of the present invention may be any timing without limitations, whether before, during, or after meals. Thus, the composition for suppressing an increase in blood glucose level of the present invention is useful as a health food, a food with function claims, or the like used for suppressing an increase in blood glucose level to prevent or ameliorate diabetes of the diabetic patient, the prediabetic patient, the borderline diabetic patient, a person concerned about postprandial hyperglycemic conditions, a healthy person, and the like through daily intake.

The composition for suppressing an increase in blood glucose level of the present invention may be labeled with function claims based on the action to suppress an increase in blood glucose level.

The composition for suppressing an increase in blood glucose level of the present invention may be labeled with one or more of the followings: "suppressing an increase in postprandial blood glucose level", "moderating an increase in postprandial blood glucose level", "reducing blood glucose level", "for those who are concerned about blood glucose level", "for those who are concerned about postprandial blood glucose level", and "reducing the predisposition for hyperglycemia".

In one embodiment of the present invention, preferably, the composition for suppressing an increase in blood glucose level of the present invention is a food or beverage labeled with one or more of the above function claims. The labels may be labels indicating use for obtaining these functions.

The present invention also encompasses a method of suppressing an increase in blood glucose level, the method including feeding or administrating isoxanthohumol to a subject.

Preferably, the method of suppressing an increase in blood glucose level is a method of suppressing an increase in postprandial blood glucose level.

The present invention encompasses a method of preventing or ameliorating a condition or disease caused by postprandial hyperglycemia, the method including feeding or administrating isoxanthohumol. Isoxanthohumol can be used for preventing or ameliorating a condition or disease caused by postprandial hyperglycemia, by suppressing an increase in postprandial blood glucose level.

The method may be a therapeutic method or a non-therapeutic method. The "non-therapeutic" is a concept that does not include medical activities, i.e., a concept that does not include surgery, therapy or diagnosis of humans.

In the method of suppressing an increase in blood glucose level or the method of preventing or ameliorating a condition or disease caused by postprandial hyperglycemia, preferably, isoxanthohumol is continuously fed or administered to a subject in order to obtain the effect of suppressing an increase in blood glucose level. The subject in the method is preferably a human or non-human mammal, more preferably a human.

The present invention also encompasses the following use:
use of isoxanthohumol for suppressing an increase in blood glucose level.

Preferably, the use is for suppressing an increase in postprandial blood glucose level.

The present invention also encompasses use of isoxanthohumol for preventing or ameliorating a condition or disease caused by postprandial hyperglycemia.

Preferably, the use is for a human or non-human mammal, more preferably a human. The use may be therapeutic or non-therapeutic use.

In the use, preferably isoxanthohumol is continuously fed or administered to a subject in order to obtain the effect of suppressing an increase in blood glucose level.

In the method and the use, when isoxanthohumol is continuously fed or administered to a subject, it is continuously fed or administered preferably for two weeks or longer, more preferably for three weeks or longer. The timing of intake or administration of isoxanthohumol is not limited. Isoxanthohumol is administered or fed to a subject in one or more portions per day, for example, one to several times (e.g., two to three times) per day. Preferably, isoxanthohumol is orally administered or fed.

In the method and the use, isoxanthohumol is administered or fed to a subject in an amount (effective amount) that provides the effect of suppressing an increase in blood glucose level. A preferred intake of isoxanthohumol, a preferred subject to be administered with isoxanthohumol, and the like are as described above for the composition for suppressing an increase in blood glucose level of the present invention. Isoxanthohumol may be directly administered or fed, or may be administered or fed in the form of a composition containing isoxanthohumol. For example, the composition for suppressing an increase in blood glucose level of the present invention may be administered or fed.

Isoxanthohumol can also be used for producing a food or beverage, a pharmaceutical or quasi-pharmaceutical product, feed, or the like used for suppressing an increase in blood glucose level. In one embodiment, the present invention also encompasses use of isoxanthohumol for producing a composition for suppressing an increase in blood glucose level.

### EXAMPLES

The following provides examples that more specifically describe the present invention. The present invention is not limited to these examples.

A series of animal experiments was performed based on a plan approved by the relevant chief through evaluation of the in-house animal experiment committee, in compliance with the animal welfare management laws and other related laws and regulations.

### <Preparation Example 1>

### Preparation of xanthohumol and isoxanthohumol

Isoxanthohumol and xanthohumol were isolated and purified from a hop extract (Asama Chemical. Co., Ltd.) by the following method. Specifically, using a hop extract as a raw material, isoxanthohumol and xanthohumol were each purified by normal-phase column chromatography, reverse phase column chromatography, and preparative HPLC, and the purity was determined to be 95% or higher by HPLC analysis. For HPLC analysis, a Develosil C30-UG-5 column (Nomura Chemical Co., Ltd.) was used. The detector wavelength to measure the UV absorption was 280 nm (isoxanthohumol) or 350 nm (xanthohumol).

The obtained isoxanthohumol and xanthohumol were used as standard samples (both having a purity of 95% or higher) in the following experiments.

### <Reference Example 1>

Evaluation of action of isoxanthohumol to suppress an increase in blood glucose level by single intake

The effect of single intake of isoxanthohumol on the increase in blood glucose level after glucose intake was examined by the following procedure.

### (Group structure)

Table 1 shows groups (group name, test substance, and dosage of the test substance) in the present test. Xanthohumol and isoxanthohumol were the standard samples (purity of 95% or higher) obtained in Preparation Example 1. The "Dosage" in the table indicates the dosage (mg) of the test substance per kg body weight. A dosing solution containing a mixture of the test substance and a solvent was used to administer the test substance. The solvent was a 0.5 wt% sodium carboxymethylcellulose (CMC) aqueous solution. The volume of the dosing solution was 10 mL/kg. A 50 wt% glucose aqueous solution was used for glucose intake.

**[Table 1]**

| Group No. | Group name | Test substance | Dosage (mg/kg) |
|---|---|---|---|
| 1 | Control | N/A | 0 |
| 2 | Xanthohumol (XN) 30 mg/kg | Xanthohumol | 30 |
| 3 | Xanthohumol (XN) 60 mg/kg | Xanthohumol | 60 |
| 4 | Isoxanthohumol (IXN) 30 mg/kg | Isoxanthohumol | 30 |
| 5 | Isoxanthohumol (IXN) 60 mg/kg | Isoxanthohumol | 60 |

### (Acclimatization and grouping)

Mice (C57BL/6J, male, 7 weeks of age, CLEA Japan, Inc.) were purchased, and they were quarantined and acclimatized for one week. Then, animals were selected based on the six-hour fasting blood glucose level from animals that showed no abnormalities in observation of general conditions. The selected animals were divided into groups (n = 9 in each group) in the group structure shown in Table 1 by the stratified continuous randomization method based on body weight at the end of the acclimatization period.

### (Glucose tolerance test)

After grouping, each group was orally administered with the test substance as described in Table 1. Thirty minutes later, each group was subjected to oral glucose intake to an amount of 1 g per 1 kg body weight, and blood was drawn from the tail vein over time. Soon after blood drawing, the blood glucose level was measured using Glutest Neo Sensor (Sanwa Kagaku Kenkyusho Co., Ltd.).

### (Statistical analysis)

Statistical processing was performed using Microsoft Office Excel 2003. A significant difference was tested between the control group and each group by Dunnett's multiple comparison test, with a significant level of 5%.

### (Results)

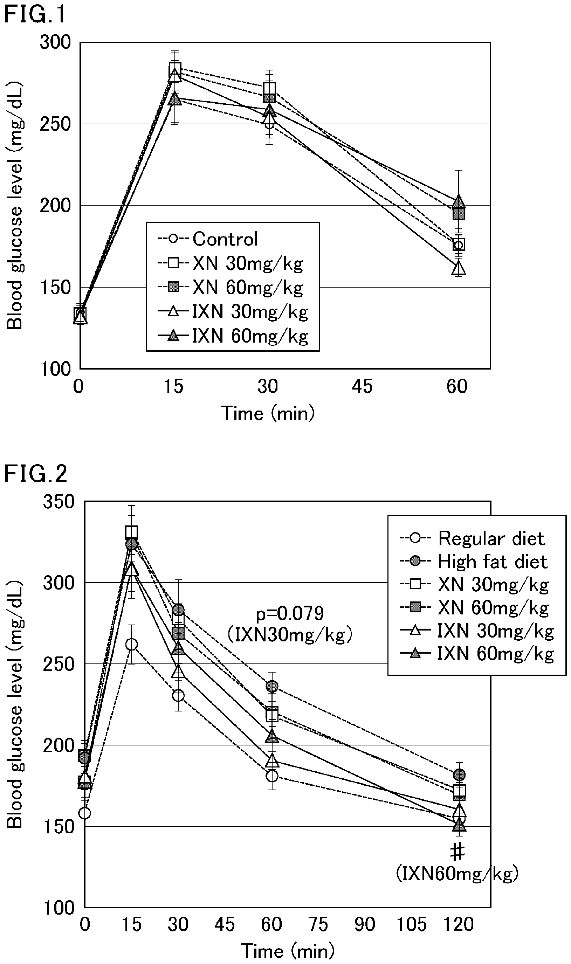

Fig. 1 is a graph showing fluctuations in blood glucose level of each group after glucose intake. The graph in Fig. 1 is presented as an average ± standard error (n = 9) of the blood glucose level of each group at each time after glucose intake (time of glucose intake: 0 minutes). In Fig. 1, XN is xanthohumol, and IXN is isoxanthohumol. In Fig. 1, ○ is a control group; □ is a xanthohumol 30 mg/kg group; ■ is a xanthohumol 60 mg/kg group; Δ is an isoxanthohumol 30 mg/kg group; and ▲ is an isoxanthohumol 60 mg/kg group.

Comparison with the control group did not show a significant action to suppress an increase in blood glucose level by single intake of xanthohumol or single intake of isoxanthohumol at any time.

### <Example 1>

Evaluation of action of isoxanthohumol to suppress an increase in blood glucose level by continuous intake

The effect of continuous intake of isoxanthohumol on the increase in blood glucose level after glucose intake was studied by the following procedure using a high-fat diet mouse model.

### (Group structure)

Table 2 shows groups (group name, basic feed, test substance, and dosage of the test substance) in the present test. In the table, "Regular diet" is control diet "D12450J" (Research Diets), and "60 kcal% High-fat diet" is very high-fat diet "D12492" (Research Diets); Xanthohumol and isoxanthohumol were the standard samples (purity of 95% or higher) obtained in Preparation Example 1. The "Dosage" in the table indicates the dosage (mg) of the test substance per day per kg body weight. As in Reference Example 1, a dosing solution containing a mixture of the test substance and a solvent (a 0.5 wt% CMC aqueous solution) was used to administer the test substance. The volume of the dosing solution was 10 mL/kg. The amount of the test substance and the volume of the solution were calculated based on the latest body weight on the administration day of the test substance. A 50 wt% glucose aqueous solution was used for glucose intake.

**[Table 2]**

| Group No. | Group name | Basic feed | Test substance | Dosage (mg/kg/day) |
|---|---|---|---|---|
| 1 | Regular diet | Regular diet | N/A | 0 |
| 2 | High fat diet | 60 kcal% High fat diet | N/A | 0 |
| 3 | Xanthohumol (XN) 30 mg/kg | 60 kcal% High fat diet | Xanthohumol | 30 |
| 4 | Xanthohumol (XN) 60 mg/kg | 60 kcal% High fat diet | Xanthohumol | 60 |
| 5 | Isoxanthohumol (IXN) 30 mg/kg | 60 kcal% High fat diet | Isoxanthohumol | 30 |
| 6 | Isoxanthohumol (IXN) 60 mg/kg | 60 kcal% High fat diet | Isoxanthohumol | 60 |

### (Acclimatization and grouping)

Mice (C57BL/6J, male, 7 weeks of age, CLEA Japan, Inc.) were purchased, and they were quarantined and acclimatized for one week. Then, animals were selected based on changes in body weight from animals that showed no abnormalities in observation of general conditions. The selected animals were divided into groups (n = 8 in each group) in the group structure shown in Table 2 by the stratified continuous randomization method based on body weight at the end of the acclimatization period. They were fed with free access to specific basic feed and water. The test substance was orally administered once a day for three weeks.

### (Glucose tolerance test)

After fasting for six hours on the last administration day of the test substance, each group was subjected to oral glucose intake to an amount of 1 g per 1 kg body weight, and blood was drawn from the tail vein over time. Soon after blood drawing, the blood glucose level was measured using Glutest Neo Sensor (Sanwa Kagaku Kenkyusho Co., Ltd.).

### (Statistical analysis)

Statistical processing was performed using Microsoft Office Excel 2003. A significant difference was tested between the regular diet group and the high fat diet group by Student's t-test (significant level: 1% (p < 0.01)). A significant difference was tested between the high fat diet group and each group excluding the regular diet group by Dunnett's multiple comparison test (significant level: 5% (p < 0.05)).

### (Results)

Fig. 2 is a graph showing fluctuations in blood glucose level of each group after glucose intake (#: p < 0.05 vs. high fat diet group). The graph in Fig. 2 is presented as an average ± standard error (n = 8) of the blood glucose level of each group at each time after glucose intake (time of glucose intake: 0 minutes). In Fig. 2, o is a regular diet group; ● is a high fat diet group; □ is a xanthohumol 30 mg/kg group; ■ is a xanthohumol 60 mg/kg group; Δ is an isoxanthohumol 30 mg/kg group; and ▲ is an isoxanthohumol 60 mg/kg group.

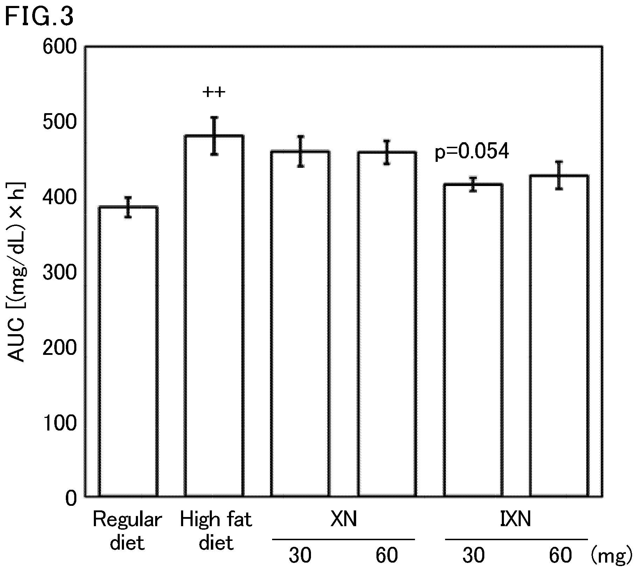

Although not shown in Fig. 2, a significant difference was found in the comparison of blood glucose level between the high fat diet group and the regular diet group at any time (p < 0.01). Fig. 3 is a graph showing an area under the time curve (AUC) of blood glucose concentration of each group (++: p < 0.01 vs. regular diet group). The results in Fig. 3 are shown as average ± standard error (n = 8) of the area under the time curve (AUC) of the blood glucose concentration from glucose intake (0 minutes) to 120 minutes later. In Fig. 2 and Fig. 3, XN is xanthohumol, and IXN is isoxanthohumol.

No significant action to suppress an increase in blood glucose level (p < 0.05) was found in either the xanthohumol 30 mg/kg group or the xanthohumol 60 mg/kg group in comparison to the high fat diet group.

A tendency for a suppressed increase in blood glucose level (p < 0.10) was found in the isoxanthohumol 30 mg/kg group in comparison to the high fat diet group, and a significant action to suppress an increase in blood glucose level was found in the isoxanthohumol 60 mg/kg group in comparison to the high fat diet group. The above shows that isoxanthohumol acts to suppress an increase in blood glucose level, and the action is more significant than that of xanthohumol.

### INDUSTRIAL APPLICABILITY

The present invention is useful in the food and beverage field and the pharmaceutical field, for example.

## Claims

1. A composition for suppressing an increase in blood glucose level, comprising:
isoxanthohumol as an active ingredient.

2. The composition for suppressing an increase in blood glucose level according to claim 1,
wherein the composition is used for suppressing an increase in postprandial blood glucose level.

3. The composition for suppressing an increase in blood glucose level according to claim 1 or 2,
wherein the composition is intended for continuous intake.

4. The composition for suppressing an increase in blood glucose level according to any one of claims 1 to 3,
wherein the composition is used for preventing or ameliorating a condition or disease caused by postprandial hyperglycemia.

5. The composition for suppressing an increase in blood glucose level according to any one of claim 1 to 4,
wherein the composition is a food or beverage.

6. The composition for suppressing an increase in blood glucose level according to any one of claims 1 to 5,
wherein the composition is a beverage.

7. The composition for suppressing an increase in blood glucose level according to claim 6,
wherein the beverage is a tea-based beverage, a coffee beverage, an alcohol beverage, a non-alcoholic beer-taste beverage, a carbonated beverage, a functional beverage, a fruit and/or vegetable-based beverage, a lactic beverage, a soy milk beverage, or flavored water.

8. The composition for suppressing an increase in blood glucose level according to any one of claims 1 to 7,
wherein the composition is labeled with one or more of the followings: "suppressing an increase in postprandial blood glucose level", "moderating an increase in postprandial blood glucose level", "reducing blood glucose level", "for those who are concerned about blood glucose level", "for those who are concerned about postprandial blood glucose level", and "reducing the predisposition for hyperglycemia".

9. A method of suppressing an increase in blood glucose level, comprising:
feeding or administering isoxanthohumol to a subject.

10. The method of suppressing an increase in blood glucose level according to claim 9,
wherein isoxanthohumol is continuously fed or administered to the subject.

11. Use of isoxanthohumol for suppressing an increase in blood glucose level.

12. The use according to claim 11,
wherein isoxanthohumol is continuously fed or administered to a subject.
